# FASCICULE DE BREVET EUROPEEN

(11) **EP 0 788 837 B1**
(45) Date de publication et mention de la délivrance du brevet: **02.05.2001**
(21) Numéro de dépôt: 97400256.0
(22) Date de dépôt: 05.02.1997
(51) Int. Cl.: B01J 29/06, C07C 6/12

(54) **Utilisation en dismutation et/ou transalkylation d'hydrocarbures alkylaromatiques d'un catalyseur composite**
Verwendung für die Disproportionierung und/oder Transalkylierung von alkylaromatischen Kohlenwasserstoffen eines zusammengesetzten Katalysators
Use for the dismutation and/or transalkylation of alkylaromatic hydrocarbons of a composite catalyst

(30) Priorité: 09.02.1996 FR 9601607
(43) Date de publication de la demande: 13.08.1997
(73) Titulaire: INSTITUT FRANCAIS DU PETROLE, 92502 Rueil-Malmaison (FR)
(72) Inventeur: Benazzi, Eric, 78360 Montesson (FR); Alario, Fabio, 92200 Neuilly sur Seine (FR)

(56) Documents cités:
- EP-A- 0 251 892
- EP-A- 0 350 367
- EP-A- 0 489 324
- FR-A- 2 644 470
- US-A- 5 210 356

## Description

L'invention concerne l'utilisation d'un catalyseur composite comprenant au moins une zéolithe de type structural mazzite, au moins en partie sous forme acide, au moins une zéolithe de type structural mordénite, au moins en partie sous forme acide, au moins une matrice et éventuellement au moins un élément choisi dans l'ensemble formé par les groupes IB et VIII de la classification périodique des éléments, en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation du toluène et d'hydrocarbures alkylaromatiques C₉⁺ pour produire des xylènes.

De nombreux catalyseurs de dismutation et de transalkylation à base de mordénite ont déjà été décrits dans l'art antérieur. Il en est ainsi dans le brevet US 3.506.731A où une mordénite sous forme hydrogène est utilisée ainsi que dans la demande de brevet français FR 2.367.533A. Il en est encore ainsi dans le brevet US 3.281.483A qui fait mention de mordénites échangées essentiellement avec des ions argent ou nickel, ou bien dans le brevet US 3.780.121A qui fait état d'une mordénite échangée avec des métaux du groupe lB de la classification périodique des éléments et qui est caractérisée par un rapport atomique Si/AI compris entre 6 et 40, ou bien encore dans le brevet US 3.629.351A qui concerne également une mordénite contenant des ions des métaux du groupe IB, VA, VIA, IIA et VIII de la classification périodique des éléments.

Plus récemment le brevet US 5.210.356A revendique l'utilisation d'un catalyseur de dismutation du toluène comportant une zéolithe oméga modifiée par désalumination et chargée en nickel.

D'autre part, le brevet US 5.371.311A nous enseigne qu'un catalyseur comportant une zéolithe oméga, synthétisée en utilisant des cations alcalins et un agent organique comme structurant organique, puis modifiée par calcination sous air, échanges ioniques, calcination en présence de vapeur d'eau et finalement par un traitement par une solution aqueuse d'ions ammonium à bas pH, conduit à des propriétés physico-chimiques supérieures et à des performances catalytiques améliorées dans les réactions de conversion des hydrocarbures et en particulier de dismutation et/ou de transalkylation des alkylaromatiques.

De façon surprenante, un catalyseur composite comprenant au moins une zéolithe de type structural mazzite, au moins en partie sous forme acide, au moins une zéolithe de type structural mordénite, au moins en partie sous forme acide, au moins une matrice et éventuellement au moins un élément choisi dans l'ensemble formé par les groupes IB et VIII de la classification périodique des éléments conduit à des performances catalytiques améliorées dans les réactions de dismutation d'hydrocarbures alkylaromatiques tel que le toluène, et/ou de transalkylation d'hydrocarbures alkylaromatiques tels que le toluène et les triméthylbenzènes, par rapport aux catalyseurs de l'art antérieur. De plus, en ajustant la quantité relative des deux zéolithes de type structural mordénite et de type structural mazzite dudit catalyseur, il devient possible de traiter une très large gamme de mélanges de charges hydrocarbonées essentiellement alkylaromatiques comportant d'une part 7 atomes de carbone par molécule et d'autre part au moins 9 atomes de carbone par molécule. Cette souplesse, qui constitue un indéniable intérêt industriel, permet ainsi de traiter des charges composites toluène-AC9(+) allant de 100% toluène à 0 % toluène - 100% AC9(+) (où AC9(+) désigne les hydrocarbures alkylaromatiques comportant au moins 9 atomes de carbone par molécule).

L'invention concerne l'utilisation d'un catalyseur composite comprenant au moins une zéolithe de type structural mordénite, de préférence la mordénite, au moins en partie, de préférence pratiquement totalement sous forme acide, au moins une zéolithe de type structural mazzite, de préférence la zéolithe oméga, au moins en partie, de préférence pratiquement totalement sous forme acide, au moins une matrice (ou liant) et éventuellement au moins un élément choisi dans l'ensemble formé par les groupes IB et VIII de la classification périodique des éléments en dismutation et/ou transalkylation du toluène et/ou d'alkylaromatiques comprenant au moins 9 atomes de carbone par molécule.

Le catalyseur composite utilisé selon l'invention contient généralement, en % poids :
- 5 à 93,99%, de préférence entre 15 et 84,95% et façon encore plus préférée entre 25 et 79,90% d'au moins une zéolithe de type structural mordénite
- 5 à 93,99%, de préférence entre 5 et 74,95%, et de façon encore plus préférée entre 5 et 59,90% d'au moins une zéolithe de type structural mazzite,
- éventuellement entre 0,01 et 10%, de préférence entre 0,05 et 7% et de façon encore plus préférée entre 0,10 et 5% d'au moins un élément choisi dans l'ensemble formé par les groupes IB et VIII de la classification périodique des éléments,
- le complément à 100 % poids consistant généralement en la part de matrice dans le catalyseur.

La matrice est choisie généralement parmi les éléments du groupe formé par les argiles (par exemple parmi les argiles naturelles telles que le kaolin ou la bentonite), la magnésie, les alumines, les silices, l'oxyde de titane, l'oxyde de bore, la zircone, les phosphates d'aluminium, les phosphates de titane, les phosphates de zirconium et les silice-alumines, de préférence parmi les éléments du groupe formé par les alumines et les argiles.

La zéolithe de type structural mazzite présente dans le catalyseur utilisé selon l'invention est généralement choisie dans le groupe formé par la zéolithe oméga, la mazzite, la zéolithe LZ-202, la zéolithe mazzite gallosilicate ou la zéolithe ZSM-4, de préférence la zéolithe oméga, dont le diamètre des pores principaux est d'environ 7,4 Å et qui posséde un réseau microporeux monodimensionnel ("Atlas of Zeolites Structure Types", W.M. Meier et D.H. Olson, 3ème Edition, 1992).

La zéolithe de type structural mazzite est au moins en partie, de préférence pratiquement totalement, sous forme acide, c'est-à-dire sous forme hydrogène (H⁺).

La zéolithe de type structural mazzite a généralement une teneur en sodium inférieure à 0,6 %, de préférence inférieure à 0,1% poids (par rapport à ladite zéolithe), comprend du silicium et au moins un élément T choisi dans le groupe formé par le gallium et l'aluminium, de préférence l'aluminium, et a un rapport molaire Si/T est compris entre 5 et 100 et de préférence entre 7 et 80.

La zéolithe de type structural mordénite présente dans le catalyseur utilisé selon l'invention est généralement choisie dans le groupe formé par la mordénite et la zéolithe LZ-211, dont le diamètre des pores principaux est d'environ 7 x 6,5 Å et qui posséde un réseau microporeux monodimensionnel ("Atlas of Zeolites Structure Types", W.M. Meier et D.H. Olson, 3ème Edition, 1992).

La zéolithe de type structural mordénite est au moins en partie, de préférence pratiquement totalement, sous forme acide, c'est-à-dire sous forme hydrogène (H⁺).

La zéolithe de type structural mordénite a généralement une teneur en sodium inférieure à 0,2 % poids, de préférence inférieure à 0,1 % poids (par rapport à ladite zéolithe), comprend du silicium et de l'aluminium et a un rapport molaire Si/AI qui est compris entre 5 et 100 et de préférence entre 7 et 80.

La zéolithe de type structural mordénite est soit à petits pores, soit à larges pores, et elle est aussi bien synthétisée en milieu hydroxyde que fluorure.

La zéolithe de type structural mordénite à petits pores possède une teneur pondérale en sodium par rapport au poids de zéolithe de type structural mordénite sèche généralement comprise entre 4 et 6,5%, un rapport atomique Si/Al global généralement compris entre 4 et 7, un volume de maille élémentaire généralement compris entre 2,76 et 2,80 nm³ (avec 1 nm=10⁻⁹m) et n'adsorbe habituellement que des molécules de diamètre cinétique inférieur à environ 4,4x10⁻¹⁰m,

La zéolithe de type structural mordénite à larges pores, synthétisée en milieu OH⁻ ou bien en milieu F⁻, par exemple selon le brevet EP 0.427.579A, se distingue de celle du type à petits pores par le fait qu'elle peut adsorber des molécules de diamètre cinétique supérieur à environ 6,6x10⁻¹⁰m, donc en particulier des molécules de benzène, que son rapport atomique Si/AI global est généralement compris entre 4,5 et 20.

Il peut être nécessaire d'amener le rapport Si/AI global de la zéolithe de type structural mordénite comprise dans le catalyseur utilisé selon l'invention à des valeurs supérieures à celles obtenues à la synthèse et mentionnées ci-avant. Afin d'obtenir des zéolithes de type structural mordénite désaluminées dans une large gamme de rapports Si/AI, toute méthode connue de l'homme du métier peut être employée comme l'attaque acide directe ou bien au moins un cycle de désalumination comprenant au moins une calcination, en présence ou non de vapeur d'eau, de la forme NH₄⁺ de la zéolithe, suivie d'au moins une attaque acide. Dans le cas spécifique de la zéolithe de type structural mordénite à petits pores, il faut s'assurer que les traitements retenus ont bien conduit à une ouverture des canaux.

Pour la préparation de la zéolithe de type structural mordénite comprise dans le catalyseur utilisé selon l'invention, la mise sous forme ammonium (NH₄⁺) s'effectue généralement soit sur une zéolithe de type structural mordénite, brute de synthèse, forme sodique, sur laquelle plusieurs échanges ioniques par des solutions concentrées de nitrate d'ammonium (10N) permettent d'obtenir une teneur en pondérale en sodium, par rapport au poids de zéolithe de type structural mordénite sèche, généralement inférieure à 2000 ppm, de préférence à 1000 ppm, et de manière encore plus préférée à 500 ppm, soit sur une zéolithe de type structural mordénite désaluminée, pour laquelle plusieurs échanges successifs au nitrate d'ammonium permettent d'obtenir la forme NH₄⁺ de ladite zéolithe.

Après ce traitement, la zéolithe de type structural mordénite modifiée subit généralement un traitement thermique destiné à décomposer au moins partiellement, de préférence pratiquement totalement, les cations ammonium présents au sein du réseau et à obtenir ainsi au moins partiellement, de préférence pratiquement totalement, la forme acide (H-M) de la zéolithe de type structural mordénite.

La zéolithe de type structural mordénite présente dans le catalyseur utilisé selon l'invention peut éventuellement avoir subi des modifications post-synthèse comme par exemple une désalumination par au moins un traitement par au moins une solution comprenant au moins un sel de fluorosilicate tel que l'hexafluorosilicate d'ammonium, comme cela est décrit dans la demande de brevet européen EP 0.573.347A ou dans le brevet US 4.503.023A, ou à l'aide d'au moins un composé des éléments choisis parmi les éléments des groupes IIA, IIB ou IVA de la classification périodique des éléments, comme cela est décrit dans la demande de brevet européen EP 0.569.268A ou dans le brevet US 5.391.528A.

La zéolithe de type structural mordénite, qu'elle soit sous forme sodium, ammonium ou hydrogène, peut être éventuellement avoir été soumise au dépôt d'au moins un élément choisi dans l'ensemble formé par les groupes IB et VIII de la classification périodique des éléments, de préférence choisi dans le groupe formé par l'argent, le nickel et le platine, et de façon encore plus préférée le nickel, et peut être mise en forme par toute technique connue de l'homme du métier.

La zéolithe de type structural mazzite présente dans le catalyseur utilisé selon la présente invention est fabriquée par toute méthode connue de l'homme du métier, à partir de zéolithe de type structural mazzite brute de synthèse dont le rapport Si/T est généralement compris entre 3 et 5.

II est généralement nécessaire d'amener le rapport Si/T de la zéolithe de type structural mazzite à une valeur supérieure à celle de la zéolithe de type structural mazzite brute de synthèse mentionnée ci-avant. Afin d'obtenir des zéolithes de type structural mazzite dans une large gamme de rapport Si/T, toute méthode connue de l'homme de l'art peut être utilisée, en particulier une désalumination selon le mode opératoire décrit dans le brevet US-A-4.780.436 dans le cas préféré où T est l'aluminium, c'est-à-dire que l'on procède à une étape de calcination sous flux d'air sec, qui a pour but d'éliminer le structurant organique occlus dans la microporosité de la zéolithe, à au moins un échange ionique par au moins une solution de NH₄NO₃, de manière à éliminer pratiquement tout cation alcalin, en particulier le sodium, présent en position cationique dans la zéolithe, puis à au moins un cycle de désalumination de la charpente, comportant au moins une calcination en présence de vapeur d'eau, à une température généralement comprise entre 550 et 850 °C, suivie d'au moins une attaque acide.

Dans le cas préféré où T est l'aluminium, la désalumination de la charpente, comportant au moins une étape de calcination sous vapeur d'eau et au moins une étape d'attaque en milieu acide de la zéolithe de type structural mazzite, peut être répété autant de fois qu'il est nécessaire pour obtenir la zéolithe de type structural mazzite désaluminée possédant les caractéristiques désirées. De même, suite au traitement de calcination sous vapeur d'eau plusieurs attaques acides successives, avec des solutions en acide de concentration différentes, peuvent être opérées.

La préparation du catalyseur utilisé selon l'invention peut être effectuée selon toute méthode connue de l'homme du métier. En général, elle est obtenue par mélange des zéolithes en poudre puis par mise en forme dudit mélange avec la matrice, ou bien, de préférence, par mise en forme des zéolithes, séparément, chacune en présence d'une partie de la matrice, comprise dans le catalyseur utilisé selon l'invention, puis le mélange des zéolithes mises en forme.

L'élément éventuel de l'ensemble formé par les groupe IB et VIII de la classification périodique des éléments peut être intoduit soit avant la mise en forme, ou bien lors du mélange, ou bien sur l'une des zéolithes avant de la mélanger, soit, de préférence, après la mise en forme. La mise en forme est généralement suivie d'une calcination, généralement à une température comprise entre 250 et 600 °C. L'élément éventuel de l'ensemble formé par les groupe IB et VIII de la classification périodique des éléments peut être intoduit après ladite calcination. Dans tous les cas, ledit élément est généralement déposé au choix soit, de préférence, pratiquement totalement sur les ou l'une des zéolithes, soit pratiquement totalement sur la matrice, soit en partie sur les ou l'une des zéolithes et en partie sur la matrice, ce choix entre le dépôt sur la matrice et le dépôt sur la phase zéolithique s'effectuant, de la manière qui est connue de l'homme du métier, par les paramètres utilisés lors dudit dépôt, comme par exemple la nature du précurseur choisi pour effectuer ledit dépôt.

L'élément des groupes IB ou VIII, de préférence choisi dans le groupe formé par Ag, Ni et Pt, et de façon encore plus préférée Ni, peut également éventuellement être déposé sur le mélange zéolithe-matrice préalablement mis en forme par tout procédé connu de l'homme de l'art. Un tel dépôt est généralement effectué par la technique d'imprégnation à sec, d'échange(s) ionique(s) ou de coprécipitation. Dans le cas de l'échange ionique à partir de précurseurs à base d'argent, de nickel ou de platine, on utilise habituellement des sels d'argent tels que les chlorures ou les nitrates, un complexe tétramine du platine, ou des sels de nickel tels que les chlorures, les nitrates, les acétates ou les formiates. Cette technique d'échange cationique peut également être utilisée pour déposer directement le métal sur la poudre de zéolithe, avant son mélange éventuel avec une matrice.

Dans le cas où le catalyseur contient plusieurs métaux, ces derniers peuvent être introduits soit tous de la même façon soit par des techniques différentes, avant ou après mise en forme et dans n'importe quel ordre. Dans le cas où la technique utilisée est celle de l'échange ionique, plusieurs échanges successifs peuvent être nécessaires pour introduire les quantités requises de métaux.

Par exemple, une des méthodes préférées de préparation du catalyseur utilisé selon l'invention consiste à malaxer les zéolithes dans un gel humide de matrice (obtenu généralement par mélange d'au moins un acide et d'une poudre de matrice), par exemple d'alumine, pendant une durée nécéssaire pour l'obtention d'une bonne homogénéité de la pâte ainsi obtenue, soit par exemple pendant une dizaine de minutes, puis à passer ladite pâte à travers une filière pour former des extrudés, par exemple de diamètre compris entre 0,4 et 4 mm. Puis, après séchage pendant quelques minutes à 100 °C en étuve et après calcination, par exemple pendant 2 heures à 400 °C, l'élément éventuel, par exemple le nickel, peut être déposé, par exemple par échange ionique, ledit dépôt étant suivi d'une calcination finale, par exemple pendant 2 heures à 400 °C.

La mise en forme du catalyseur utilisé selon l'invention est généralement telle que le catalyseur est de préférence sous forme de pastilles, d'aggrégats, d'extrudés ou de billes, en vue de son utilisation.

La préparation du catalyseur se termine généralement toujours par une calcination, dite calcination finale, généralement à une température comprise entre 250 et 600 °C, de préférence précédée d'un séchage, par exemple à l'étuve, à une température généralement comprise entre la température ambiante et 250 °C, de préférence entre 40 et 200 °C. Ladite étape de séchage est de préférence menée pendant la montée en température nécessaire pour effectuer ladite calcination.

Le dépôt éventuel de l'élément (ou des éléments) des groupes IB et VIII est suivi en général d'une calcination sous air ou oxygène, généralement entre 300 et 600°C, de préférence entre 350 et 550°C, et pour une durée comprise entre 0,5 et 10 heure(s), de préférence entre 1 et 4 heure(s). On peut procéder ensuite à une réduction sous hydrogène, généralement à une température comprise entre 300 et 600°C, de préférence entre 350 et 550°C, et pour une durée comprise entre 1 et 10 heure(s), de préférence entre 2 et 5 heures, de façon à obtenir ledit élément principalement sous forme réduite nécessaire à l'activité catalytique.

L'invention concerne aussi l'utilisation dudit catalyseur en dismutation d'hydrocarbures alkylaromatiques, et de préférence la dismutation du toluène pour produire du benzène et des xylènes, et/ou en transalkylation d'hydrocarbures alkylaromatiques, et de préférence la transalkylation d'hydrocarbures alkylaromatiques généralement en C₉⁺ (c'est-à-dire à au moins 9 atomes de carbone par molécule), telle que la transalkylation et/ou la dismutation du toluène et/ou d'alkylaromatiques C₉⁺ pour produire des xylènes. La charge d'un tel procédé peut comprendre de 0 à 100 % d'alkylaromatiques en C₉⁺ et de 0 à 100 % de toluène.

Les conditions opératoires sont généralement les suivantes : une température comprise entre 250 et 650°C et de préférence entre 350 et 550°C ; une pression comprise entre 5 et 60 bar et de préférence entre 15 et 45 bar ; une vitesse spatiale d'alimentation, exprimée en kilogramme de charge introduite par kilogramme de catalyseur et par heure, comprise entre 0,1 et 10 et de préférence entre 0,5 et 5 ; un rapport molaire hydrogène sur hydrocarbures compris entre 2 et 20 et de préférence entre 3 et 12.

Les exemples suivants illustrent l'invention sans toutefois en limiter la portée.

### Exemple 1: Préparation du catalyseur M1

La matière première utilisée est une mordénite, qui possède un rapport atomique Si/AI global égal à 7,6, une teneur pondérale en sodium par rapport au poids en mordénite sèche d'environ 3,8%, un volume de maille élémentaire de 2,759 nm³, et un volume poreux, à l'azote, mesuré à -196°C et à P/Pₒ=0,19 de 0,192 cm³ liquide par gramme.

Cette mordénite subit une attaque acide, à l'aide d'une solution d'acide nitrique 3N, à environ 100°C, pendant 4 heures, de manière à extraire partiellement les atomes d'aluminium présent dans la mordénite. Le volume V de la solution d'acide nitrique engagé (en ml) est égal à 10 fois le poids P de mordénite sèche (V/P=10). La mordénite ainsi désaluminée est ensuite soumise à un échange ionique dans une solution de NH₄NO₃ 10N à environ 100°C pendant 4 heures de manière à retirer le sodium résiduel.

A l'issue de ces traitements, la mordénite sous forme H a un rapport Si/AI atomique global égal à 20,6, une teneur pondérale en sodium par rapport au poids de mordénite sèche de 65 ppm, un volume de maille élémentaire de 2,726 nm³, et une capacité d'adsorption en azote mesurée à -196°C et à P/Pₒ=0,19 de 0,21 cm³ liquide/g.

Cette mordénite est ensuite mise en forme par extrusion avec un gel d'alumine de manière à obtenir, après séchage et calcination sous air sec, le catalyseur **M1** qui contient en % poids 80,2 % de mordénite forme hydrogène et 19,8 % d'alumine.

### Exemple 2 : Préparation du catalyseur Ω1

La matière première utilisée est une zéolithe oméga, qui possède un rapport atomique Si/AI global égal à 3,2, une teneur pondérale en sodium par rapport au poids en zéolithe oméga sèche d'environ 5,3 %, un volume de maille élémentaire de 2,196 nm³, et un volume poreux, à l'azote, mesuré à -196°C et à P/Pₒ=0,19 de 0,125 cm³ liquide par gramme.

Cette zéolithe oméga subit tout d'abord une calcination dite sèche à 550°C sous flux d'air et d'azote durant 6 heures. Puis le solide obtenu est soumis à trois échanges ioniques dans une solution de NH₄NO₃ 10N, à environ 100°C pendant 4 heures pour chaque échange. La zéolithe oméga est alors soumise à un traitement hydrothermique, en présence de 50% de vapeur d'eau à 600°C, durant 4 heures. La zéolithe subit alors une attaque acide, à l'aide d'une solution d'acide nitrique 1N, à environ 100°C, pendant 2 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10). Puis la zéolithe oméga précédemment traitée est soumise de nouveau à un traitement hydrothermique, en présence de 50% de vapeur d'eau mais cette fois ci à à 700°C, durant 4 heures, puis à une attaque acide, par une solution d'acide nitrique 1,5N à environ 100°C, pendant 4 heures, de manière à extraire les espèces aluminiques extra-réseau formées lors du traitement hydrothermique. Le volume V de la solution d'acide nitrique engagé (en ml) est égal à 10 fois le poids P de zéolithe oméga sèche (V/P=10).

A l'issue de ces traitements, la zéolithe oméga sous forme H a un rapport Si/Al atomique global égal à environ 25, une teneur pondérale en sodium par rapport au poids de zéolithe oméga sèche de 90 ppm, un volume de maille élémentaire de 2,115 nm³, et une capacité d'adsorption en azote mesurée à -196°C et à P/Pₒ=0,19 de 0,206 cm³ N2 liquide/g.

Cette zéolithe oméga est ensuite mise en forme par extrusion avec un gel d'alumine de manière à obtenir, après séchage et calcination sous air sec, le catalyseur Ω**1** qui contient en % poids 80,2 % de zéolithe oméga et 19,8 % d'alumine.

### Exemple 3 : Préparation du catalyseur M2

Le catalyseur M1 obtenu à l'exemple 1, est soumis à 3 échanges ioniques avec une solution d'acétate de nickel de manière à introduire 1,0% poids de nickel dans le catalyseur.

Pour cela, le catalyseur M1 est mis en contact avec une solution 0,5 M de Ni(CH₃CO₂)₂ à température ambiante et sous agitation. Entre chaque échange, le solide est séparé de la solution d'imprégnation et lavé abondamment à l'eau permutée. La concentration de la solution d'imprégnation est pour chaque échange ré-ajustée à la concentration de 0,5 mole par litre.

Le solide humide est ensuite séché à 120°C pendant 12 heures, et calciné sous un débit d'air sec à la température de 500°C pendant 1 heure. Le catalyseur **M2** ainsi obtenu contient, en % poids, 79,3 % de mordénite forme hydrogène, 19,6 % d'alumine et 1,1 % de nickel.

### Exemple 4 : Préparation du catalyseur Ω2

Le catalyseur Ω**1** obtenu à l'exemple 2, est soumis à 3 échanges ioniques avec une solution d'acétate de nickel de manière à introduire 1,0% poids de nickel dans le catalyseur.

Les conditions opératoires des échanges ioniques, du lavage, du séchage et de la calcination sont identiques à celles indiquées dans l'exemple 3.

Le catalyseur Ω**2** ainsi obtenu contient, en % poids, 79,5 % de zéolithe oméga, 19,6 % d'alumine et 0,9 % de nickel.

### Exemple 5 : Préparation des catalyseurs A et B conformes à l'invention

Le catalyseur A est obtenu en mélangeant les catalyseurs M1 de l'exemple 1 et Ω**1** de l'exemple 2 avec les pourcentages pondéraux suivants : 86 % de M1 et 14 % de Ω**1**, de sorte que le catalyseur A contienne, en % poids, 69,0 % de mordénite, 11,2 % de zéolithe oméga, et 19,8 % d'alumine.

Selon le même principe, le catalyseur B est le résultat du mélange des catalyseurs M2 de l'exemple 3 et Ω**2** de l'exemple 4 avec les pourcentages pondéraux suivants : 86 % de M2 et 14 % de Ω**2**. Le catalyseur B contient alors, en % poids, 68,2 % de mordénite, 11,1 % de zéolithe oméga, 1,1 % de nickel, et 19,6 % d'alumine.

Un synopsis des compositions des catalyseurs M1 de l'exemple 1, et Ω**1** de l'exemple 2, M2 de l'exemple 3, Ω**2** de l'exemple 4, A et B de l'exemple 5, est donné dans le tableau suivant.

| Catalyseurs | Zéolithe **Mordénite** (% poids) | Zéolithe **Oméga** (% poids) | **Nickel** (% poids) | **Alumine** (% poids) |
|---|---|---|---|---|
| **M1** | 80,2 | 0,0 | 0,0 | 19,8 |
| Ω**1** | 0,0 | 80,2 | 0,0 | 19,8 |
| **A** selon l'invention (86 %M1 + 14% Ω**1** ) | 69,0 | 11,2 | 0,0 | 19,8 |
| **M2** | 79,3 | 0,0 | 1,1 | 19,6 |
| Ω**2** | 0,0 | 79,5 | 0,9 | 19,6 |
| **B** selon l'invention (86 %M2 + 14% Ω**2**) | 68,2 | 11,1 | 1,1 | 19,6 |

### Exemple 6 : Evaluation des performances des catalyseurs

Les catalyseurs sont mis en oeuvre dans un réacteur à lit fixe, sous pression, dans lequel est introduit la charge qui est constituée de toluène pur.

La comparaison des rendements en (benzène + ethylbenzène + xylènes) obtenus en utilisant les catalyseurs A et B, conformes à l'invention, et M1, M2, Ω1 et Ω2 non conformes à l'invention, est effectuée dans le tableau ci-après :

| Catalyseurs | M1 (non conforme) | Ω1 (non conforme) | A (conforme) | M2 (non conforme) | Ω2 (non conforme) | B (conforme) |
|---|---|---|---|---|---|---|
| Température de réaction (°C) | 450 | 450 | 450 | 430 | 430 | 430 |
| Pression totale de réaction(Bar) | 30 | 30 | 30 | 40 | 40 | 40 |
| Rendements % poids (Benzène + Ethylbenzène + xylènes) | 43,2 | 38,9 | 43,4 | 42,1 | 36,9 | 42,4 |

La comparaison des catalyseurs A et B et d'autre part la comparaison des catalyseurs M1, M2, Ω1 et Ω2, montre que les catalyseurs selon l'invention, A et B, conduisent à des rendements en (Benzène + Ethylbenzène + xylènes) supérieurs à ceux obtenus avec les catalyseurs non conformes M1, M2, Ω1 et Ω2.

## Revendications

1. Utilisation en dismutation d'hydrocarbures alkylaromatiques et/ou en transalkylation d'hydrocarbures alkylaromatiques d'un catalyseur composite comprenant au moins une zéolithe de type structural mordénite au moins en partie sous forme acide, au moins une zéolithe de type structural mazzite au moins en partie sous forme acide, au moins une matrice et éventuellement au moins un élément choisi dans l'ensemble formé par les groupes IB et VIII de la classification périodique des éléments.

2. Utilisation selon la revendication 1 telle que le catalyseur contient, en % poids :
- 5 à 93,99% d'au moins une zéolithe de type structural mordénite
- 5 à 93,99% d'au moins une zéolithe de type structural mazzite,
- éventuellement entre 0,01 et 10% d'au moins un élément choisi dans l'ensemble formé par les groupes IB et VIII de la classification périodique des éléments,
- le complément à 100 % poids consistant en la part de matrice dans le catalyseur.

3. Utilisation selon l'une des revendications 1 ou 2 telle que la matrice est choisie dans le groupe formé par les alumines, la magnésie, les silices, les silice-alumine et les argiles.

4. Utilisation selon l'une des revendications 1 à 3 telle que ledit élément est choisi dans le groupe formé par Ag, Pt et Ni.

5. Utilisation selon l'une des revendications 1 à 4 telle que la zéolithe de type structural mazzite est choisie dans le groupe formé par la zéolithe mazzite gallosilicate, la mazzite, la zéolithe LZ-202, la zéolithe oméga ou la zéolithe ZSM-4.

6. Utilisation selon l'une des revendications 1 à 5 telle que la zéolithe de type structural mazzite est la zéolithe oméga.

7. Utilisation selon l'une des revendications 1 à 6 telle que la zéolithe de type structural mazzite est pratiquement totalement sous forme acide.

8. Utilisation selon l'une des revendications 1 à 7 telle que la zéolithe de type structural mazzite a une teneur en sodium inférieure à 0,6% poids (par rapport à ladite zéolithe), comprend du silicium et de l'aluminium, et a un rapport molaire Si/Al qui est compris entre 5 et 100.

9. Utilisation selon l'une des revendications 1 à 8 telle que la zéolithe de type structural mordénite est choisie dans le groupe formé par la mordénite, la zéolithe LZ-211.

10. Utilisation selon l'une des revendications 1 à 9 telle que ladite zéolithe de type mordénite est la mordénite.

11. Utilisation selon l'une des revendications 1 à 10 telle que la zéolithe de type structural mordénite est pratiquement totalement sous forme acide.

12. Utilisation selon l'une des revendications 1 à 11 tel que la zéolithe de type structural mordénite a une teneur en sodium inférieure à 0,2% poids (par rapport à ladite zéolithe), comprend du silicium et de l'aluminium, et a un rapport molaire Si/Al qui est compris entre 5 et 100.

13. Utilisation selon l'une des revendications 1 à 12 telle que le catalyseur est sous forme de pastilles, d'aggrégats, d'extrudés ou de billes.

14. Utilisation du catalyseur selon l'une des revendications 1 à 13 en dismutation et/ou en transalkylation de toluène et/ou d'alkylaromatiques comprenant au moins 9 atomes de carbone par molécule.

## Patentansprüche

1. Verwendung eines Verbundkatalysators bei der Dismutation von alkylaromatischen Kohlenwasserstoffen und/oder bei der Transalkylierung von alkylaromatischen Kohlenwasserstoffen, der zumindest einen Zeolith vom Mordenitstrukturtyp, der zumindest teilweise in saurer Form ist, zumindest einen Zeolith vom Mazzitstrukturtyp, der zumindest teilweise in saurer Form ist, zumindest eine Matrix und gegebenenfalls zumindest ein Element umfasst, das in der durch die Gruppen IB und VIII des Periodensystems der Elemente gebildeten Gesamtheit gewählt ist.

2. Verwendung nach Anspruch 1 derart, dass der Katalysator an Gew.-% enthält:
- 5 bis 93,99% wenigstens eines Zeoliths vom Mordenitstrukturtyp
- 5 bis 93,99% zumindest eines Zeoliths vom Mazzitstrukturtyp;
- gegebenenfalls zwischen 0,01 und 10% wenigstens eines in der durch die Gruppen IB und VIII des Periodensystems der Elemente gebildeten Gesamtheit gewählten Elements,
- die Ergänzung auf 100 Gew.-%, welche aus dem Matrixteil in dem Katalysator besteht.

3. Verwendung nach einem der Ansprüche 1 oder 2 derart, dass die Matrix in der durch die Aluminiumoxide, Magnesiumoxid, den Siliziumoxiden, den Silizium-Aluminiumoxiden und den Tonen gebildeten Gruppe gewählt ist.

4. Verwendung nach einem der Ansprüche 1 bis 3 derart, dass dieses Element aus der durch Ag, Pt und Ni gebildeten Gruppe gewählt ist.

5. Verwendung nach einem der Ansprüche 1 bis 4 derart, dass der Zeolith vom Mazzitstrukturtyp in der durch den Zeolith Mazzit-Gallosilikat, Mazzit, den Zeolith LZ-202, den Omegazeolith oder den Zeolith ZSM-4 gebildeten Gruppe gewählt ist.

6. Verwendung nach einem der Ansprüche 1 bis 5 derart, dass der Zeolith vom Strukturtyp Mazzit der Omegazeolith ist.

7. Verwendung nach einem der Ansprüche 1 bis 6 derart, dass der Zeolith vom Strukturtyp Mazzit praktisch vollständig in saurer Form vorliegt.

8. Verwendung nach einem der Ansprüche 1 bis 7 derart, dass der Zeolith vom Mazzitstrukturtyp einen Natriumgehalt kleiner als 0,6 Gew.-% (im Verhältnis zu diesem Zeolith) hat, Silizium und Aluminium umfasst und ein molares Si/Al-Verhältnis hat, das zwischen 5 und 100 liegt.

9. Verwendung nach einem der Ansprüche 1 bis 8 derart, dass der Zeolith vom Strukturtyp Mordenit in der durch Mordenit, den Zeolith LZ-211 gebildeten Gruppe gewählt ist.

10. Verwendung nach einem der Ansprüche 1 bis 9 derart, dass dieser Zeolith vom Mordenittyp Mordenit ist.

11. Verwendung nach einem der Ansprüche 1 bis 10 derart, dass der Zeolith vom Strukturtyp Mordenit praktisch vollständig in saurer Form ist.

12. Verwendung nach einem der Ansprüche 1 bis 11 derart, dass der Zeolith vom Mordenitstrukturtyp einen Natriumgehalt kleiner als 0,2 Gew.-% (im Verhältnis zu diesem Zeolith) hat, Silizium und Aluminium umfasst und ein molares Si/Al-Verhältnis hat, das zwischen 5 und 100 liegt.

13. Verwendung nach einem der Ansprüche 1 bis 12 derart, dass der Katalysator in Form von Tabletten, Aggregaten, Extrudaten oder Kugeln vorliegt.

14. Verwendung des Katalysators nach einem der Ansprüche 1 bis 13 bei der Dismutation und/oder bei der Transalkylierung von Toluol und/oder Alkylaromaten, die wenigstens 9 Kohlenstoffatome pro Molekül umfassen.

## Claims

1. The use of a composite catalyst comprising at least one zeolite with structure type mordenite, at least partially in its acid form, at least one zeolite with structure type mazzite, at least partially in its acid form, at least one matrix and, optionally, at least one element selected from the group formed by groups IB and VIII of the periodic classification of the elements, for the dismutation of alkylaromatic hydrocarbons and/or for the transalkylation of alkylaromatic hydrocarbons.

2. Use according to claim 1, in which the catalyst contains, by weight:
• 5% to 93.99% of at least one zeolite with structure type mordenite;
• 5% to 93.99% of at least one zeolite with structure type mazzite;
• optionally, between 0.01% and 10% of at least one element selected from the group formed by groups IB and VIII of the periodic classification of the elements;
• the complement to 100% consisting of the catalyst matrix.

3. Use according to claim 1 or claim 2, in which the matrix is selected from the group formed by aluminas, magnesia, silicas, silica-aluminas and clays.

4. Use according to any one of claims 1 to 3, in which said element is selected from the group formed by Ag, Pt and Ni.

5. Use according to any one of claims 1 to 4, in which the zeolite with structure type mazzite is selected from the group formed by gallosilicate mazzite zeolite, mazzite, LZ-202 zeolite, omega zeolite, and ZSM-4 zeolite.

6. Use according to any one of claims 1 to 5, in which the zeolite with structure type mazzite is omega zeolite.

7. Use according to any one of claims 1 to 6, in which the zeolite with structure type mazzite is practically completely in its acid form.

8. Use according to any one of claims 1 to 7, in which the zeolite with structure type mazzite has a sodium content of less than 0.6% by weight (with respect to said zeolite), contains silicon and aluminium, and has an Si/Al molar ratio which is in the range 5 to 100.

9. Use according to any one of claims 1 to 8, in which the zeolite with structure type mordenite is selected from the group formed by mordenite, and LZ-211 zeolite.

10. Use according to any one of claims 1 to 9, in which said zeolite with structure type mordenite is mordenite.

11. Use according to any one of claims 1 to 10, in which the zeolite with structure type mordenite is practically completely in its acid form.

12. Use according to any one of claims 1 to 11, in which the zeolite with structure type mordenite has a sodium content of less than 0.2% by weight (with respect to said zeolite), contains silicon and aluminium, and has an Si/Al molar ratio which is in the range 5 to 100.

13. Use according to any one of claims 1 to 12, in which the catalyst is in the form of pellets, aggregates, extrudates or spherules.

14. Use according to any one of claims 1 to 13, for the dismutation and/or transalkylation of toluene and/or alkylaromatics containing at least 9 carbon atoms per molecule.
